(19) Europäisches Patentamt
European Patent Office
Office européen des brevets

(11) Veröffentlichungsnummer: **0 125 533**
**B1**

(12) # EUROPÄISCHE PATENTSCHRIFT

(45) Veröffentlichungstag der Patentschrift: **11.06.86**

(51) Int. Cl.⁴: **C 07 C 69/675**, C 07 C 67/22

(21) Anmeldenummer: **84104564.4**

(22) Anmeldetag: **24.04.84**

(54) **Verfahren zur Herstellung von Tartronsäureestern.**

(30) Priorität: **04.05.83 DE 3316264**

(43) Veröffentlichungstag der Anmeldung: **21.11.84 Patentblatt 84/47**

(45) Bekanntmachung des Hinweises auf die Patenterteilung: **11.06.86 Patentblatt 86/24**

(84) Benannte Vertragsstaaten: **CH DE FR GB IT LI**

(56) Entgegenhaltungen:
**EP - A - 0 056 264**
**DE - A - 2 524 222**

(73) Patentinhaber: **BAYER AG, Konzernverwaltung RP Patentabteilung, D-5090 Leverkusen 1 Bayerwerk (DE)**

(72) Erfinder: **Findeisen, Kurt, Dr., In der Follmühle 10, D-5068 Odenthal 2 (DE)**
Erfinder: **Fauss, Rudolf, Dr., Gerstenkamp 10, D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1986

**Beschreibung**

Die Erfindung betrifft ein neues Verfahren zur Herstellung von Tartronsäuralkyl- und -cycloalkylestern.

Tartronsäurealkylester, z.B. Tartronsäuremethyl- und -ethylester wurden bislang durch Umsetzung der Natrium-, Calcium-, oder Barium-Salze der Tartronsäure mit dem entsprechenden wasserfreien, Chlorwasserstoff enthaltenden Alkohol erhalten (siehe die Angaben in Beilsteins Handbuch der Organischen Chemie, Bd. 3, S. 416, 1. Ergänzungswerk S. 148, 2. Ergänzungswerk S. 274.

Wegen der schwierigen Zugänglichkeit der Tartronsäure stellt diese Umsetzung jedoch kein eigentliches Herstellungsverfahren sondern lediglich eine Bildungweise dar. Da Tartronsäureester jedoch als Ausgangsverbindungen für die Herstellung von Fungiziden der N-Aryl-1,3-oxazolidin-2,4-dion-Reihe praktische Verwendung gefunden haben (siehe DE-OS 29 06 574) bestand die technische Aufgabe, ein einfaches, wirtschaftliches Herstellungsverfahren für die Herstellung von Tartronsäureestern aufzufinden.

Erfindungsgemäß wurde gefunden, daß man in einfacher und wirtschaftlicher Weise Tartronsäurealkyl- und cycloalkylester der Formel

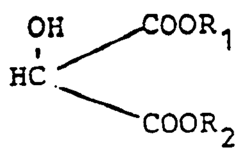

(I)

erhält, wenn man vom Trimethylsilyloxy-tricyan-methan der Formel

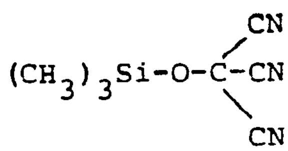

(II)

ausgeht und dieses in einer zweistufigen Reaktion zunächst mit Alkoholen der Formel $R_1OH$ und $R_2OH$ in Gegenwart von Mineralsäuren umsetzt und anschließend das Umsetzungsprodukt hydrolysiert. Überraschenderweise wurde gefunden, daß die Umsetzung des Trimethylsilyloxytricyan-methans nach pinner, nicht wie zu erwarten zur Umwandlung aller CN-Gruppen in COOR-Gruppen führt, sondern, daß bei der Anwendung der primer-Reaktion auf das Trimethylsilyloxy-tricyan-methan eine der CN-Gruppen durch Wasserstoff ersetzt wird.

Die Erfindung betrifft daher ein Verfahren zur Herstellung von Tartronsäureestern der Formel

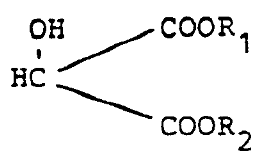

(I)

in der $R_1$ und $R_2$ unabhängig voneinander für einen gegebenen falls substituierten Alkyl- oder Cycloalkyl-Rest stehen,

das dadurch gekennzeichnet ist, daß man (Trimethylsilyloxy)-tricyan-methan mit Alkoholen der Formel $R_1OH$ und $R_2OH$ in Gegenwart von Mineralsäuren umsetzt und das Umsetzungsprodukt hydrolysiert.

Bevorzugt sind die Ester der Formel (I), in der $R_1$ und $R_2$ unabhängig voneinander für einen $C_1$-$C_8$-Alkyl- oder einen $C_3$-$C_6$-Cycloalkyl-Rest stehen.

Das erfindungsgemäße Verfahren gestattet auch die Herstellung von Tartronsäurealkyl- und -cycloalkylestern der Formel (I), deren Alkyl- bzw. Cycloalkyl-Reste $R_1$ und $R_2$ z.B. durch Halogenatome, insbesondere Fluor-, Chlor- und Brom-Atome, $C_1$-$C_4$-Alkyl-Reste, $C_1$-$C_4$-Alkoxy-Reste z.B. den Methoxy- oer den Ethoxy-Rest, und/oder phenyl- oder Phenoxy-Reste substituiert sind. Beispiele solcher substituiertes Alkylreste sind z.B. der 2-Fluor-, 2-Methoxy-, 2-Ethoxy- und 2-Phenoxyethylrest, ferner der Methyl- und der tert.-Butylcyclohexylrest.

Vorzugsweise stehen $R_1$ und $R_2$ für den Methyl, Ethyl, n-propyl und den Isopropyl-Rest.

Das erfindungsgemäß als Ausgangsverbindung zu verwendende (Trimethylsilyloxy)-tricyano-methan ist

bekannt und wird durch Umsetzung von phosgen mit Trimethylsilylcyanid erhalten (siehe Angewandte Chemie 94, 4 (1982)).

Bei der Ausführung des erfindungsgemäßen Verfahrens ist eine Isolierung des bei der Umsetzung des (Trimethylsilyloxy)-tricyan-methans mit den Alkoholen in Gegenwart der Mineralsäuren entstehenden Umsetzungsproduktes nicht erforderlich. Umsetzung und Hydrolyse werden vielmehr - vorzugsweise - unmittelbar hintereinander in ein und demselben Reaktionsgefäß vorgenommen.

Für die Umsetzung der (Trimethylsilyloxy)-tricyan-methans werden 3 Mol Alkohol und 3 Äquivalente Mineralsäure je Mol (Trimethylsilyloxy)-tricyan-methan benötigt. Vorteilhaft verwendet man jedoch einen Überschuß am Alkohol, z.B. 4 bis 20 Mol Alkohol je Mol (Trimethylsilyloxy)-tricyanmethan; der überschüssige Alkohol dient zugleich als Verdünnungsmittel. Anstelle überschüssiger Alkohole können auch unter den Reaktionsbedingungen inerte organische Verdünnungsmittel beispielsweise Kohlenwasserstoffe wie Benzol und Toluol, oder halogenierte Kohlenwasserstoffe wie Methylenchlorid, Dichlorethan und Chlorbenzol oder Sulfolan verwendet werden.

Als Mineralsäuren werden vor allem Schwefelsäure, Phosphorsäure und perchlorsäure und - vorzugsweise - Chlorwasserstoff verwendet.

Die erfindungsgemäße Umsetzung des (Trimethylsilyloxy)tricyan-methans mit den Alkoholen in Gegenwart der Mineralsäuren wird im allgemeinen bei Temperaturen von -10 bis 60° C, vorzugsweise von 10 bis 40° C vorgenommen. Zur Vervollständigung der Reaktion wird das Reaktionsgemisch bei Temperaturen von 20 bis 80° C nachgerührt und anschließend mit einer solchen Wassermenge versetzt, daß mindestens 3 Mol Wasser auf 1 Mol (Trimethylsilyloxy)tricyan-methan entfallen. Ein Überschuß an Wasser über diese stöchiometrisch erforderliche Menge hinaus stört nicht. Nach der Wasserzugabe wird die Reaktionsmischung bei Temperaturen von 20° C bis zum Siedepunkt der Reaktionsmischung nachgerührt und anschließend in an sich bekannter Weise aufgearbeitet. Die Aufarbeitung des Reaktionsgemisches kann unmittelbar durch Destillation erfolgen; bei dieser Arbeitsweise wird vorteilhaft zunächst das ausgefallene Ammoniumchlorid abfiltriert. Die Aufarbeitung kann aber auch in der Weise erfolgen, daß man das Reaktionsgemisch zunächst auf Eis(wasser) austrägt und den Ester durch Extraktion mit einem weitgehend in Wasser unlöslichen Lösungsmittel, z.B. Methylenchlorid, Essigsäuremethylester, Toluol oder Chlorbenzol von der wäßrigen phase abtrennt und erst den Extrakt destillativ aufarbeitet. Bevorzugt wird die Veresterung mit Salzsäure und die unmittelbare destillative Aufarbeitung angewendet. Das als Nebenprodukt bei der Reaktion in untergeordneten Mengen entstehende 0-substi-tuierte Urethan läßt sich durch Destillation vom gewünschten Tartronsäureester abtrennen.

Die erfindungsgemäße Umsetzung des (Trimethylsilyloxy)tricyan-methans mit den Alkoholen in Gegenwart der Mineralsäuren wird vorzugsweise in der Weise vorgenommen, daß man den Alkohol mit der anorganischen Säure vorlegt und das (Trimethylsilyloxy)-tricyan-methan unter Rühren einträgt. Die Umsetzung kann aber auch in der Weise vorgenommen werden, daß man das (Trimethylsilyloxy)tricyan-methan zunächst zur Mineralsäure gibt und anschließend den wasserfreien Alkohol zusetzt. Wird die Umsetzung unter Verwendung nur eines Alkohols durchgeführt so erhält man Tartronsäureester mit gleichen Esterresten. Für die Herstellung von gemischten Tartronsäureestern werden Gemische der Estergruppen bildenden Alkohole eingesetzt. Am besten hat sich die Arbeitsweise bewährt, bei der der wasserfreie Alkohol mit Chlorwasserstoffgas gesättigt wird und (Trimethylsilyloxy)tricyan-methan in diesen salzsauren Alkohol eingetragen wird.

**Beispiel 1**

1800 ml absolutes Ethanol wird bei Raumtemperatur mit HCl-Gas gesättigt und anschließend bei höchstens 30° C zunächst mit 537 g (Trimethylsilyloxy)-tricyan-methan und nach 30-minütigem Rühren mit 54 g Wasser tropfenweise versetzt. Nach 1-stündigem Erwärmen auf Rückflußtemperatur und nachfolgendem Abkühlen wird das $NH_4Cl$ abgesaugt und das Filtrat im Vakuum eingeengt. Der flüssige Rückstand wird im Vakuum (über eine 1 Meter lange Vigreux-Kolonne) destilliert.

Es werden 316 g (55 % der Theorie) Tartronsäurediethylester vom Kp 111-113° C/16 mbar erhalten.

**Beispiel 2**

300 ml wasserfreies Ethanol wird bei 10° C mit HCl-Gas gesättigt und anschließend bei 0° C mit 102 g (Trimethylsilyloxy)-tricyan-methan versetzt. Die Mischung wird 1 Stunde bei 15° C nachgerührt und anschließend mit 300 ml Wasser verdünnt. Nach 30-minütigem Nachrühren wird mit Eiswasser versetzt und mit Methylenchlorid extrahiert. Die Aufarbeitung des Methvlenchlorid-Extraktes und die Vakuumdestillation des Rückstandes ergeben 86 g (50 % der Theorie) Tartronsäurediethylester vom Kp 112-113° C/16 mbar.

**Beispiel 3**

Eine Mischung aus 200 g (100 %ige) Schwefelsäure und 50 g Methanol wird bei 30°C tropfenweise unter Rühren mit 44 g (Trimethylsilyloxy)-tricyan-methan versetzt. Die Temperatur wird während der Zugabe auf 50 bis 60°C gehalten. Anschließend wird das Reaktionsgemisch mit einer Mischung aus 13,5 g Wasser und 50 ml Methanol versetzt und 1 Stunde bei 40°C weitergerührt. Die Reaktionsmischung wird auf Eis gegossen und mehrmals mit Methylenchlorid extrahiert. Die vereinigten Methylenchlorid-Extrakte werden einmal mit wenig Wasser gewaschen und anschließend eingeengt. Bei der Destillation des Rohproduktes wurden 10 g (31 % der Theorie) Tartronsäuredimethylester vom Kp 112°C/20 mbar, Fp: 55°C erhalten.

**Patentansprüche**

1. Verfahren zur Herstellung von Tartronsäureestern der Formel

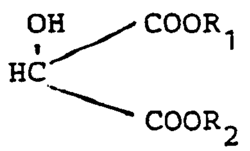

(I)

in der
$R_1$ und $R_2$ unabhängig voneinander für einen gegebenenfalls substituierten Alkyl- oder Cycloalkyl-Rest stehen, dadurch gekennzeichnet, daß man (Trimethylsilyloxy)tricyan-methan mit Alkoholen der Formel $R_1OH$ und $R_2OH$, in denen $R_1$ und $R_2$ die vorstehend angegebene Bedeutung haben, in Gegenwart von Mineralsäuren umsetzt und das Umsetzungsprodukt hydrolysiert.

2. Verfahren nach Anspruch 1, dadurch gekennzeichnet, daß man die Alkohole $R_1OH$ und $R_2OH$ in einer Menge von mindestens 3 Mol je Mol (Trimethylsilyloxy)tricyan-methan einsetzt.

3. Verfahren nach Anspruch 1 und 2, dadurch gekennzeichnet, daß man die Umsetzung des (Trimethylsilyloxy)-tricyan-methans in einem Verdünnungsmittel vornimmt.

4. Verfahren nach Anspruch 3, dadurch gekennzeichnet, daß man überschüssigen Alkohol $R_1OH$ und $R_2OH$ als Verdünnungsmittel verwendet.

5. Verfahren nach Anspruch 1 bis 4, dadurch gekennzeichnet, daß man die Umsetzung des (Trimethylsilyloxy)-tricyan-methans bei Temperaturen von −10 bis +60°C vornimmt.

6. Verfahren gemäß Anspruch 1 bis 5, dadurch gekennzeichnet, daß man die Umsetzung des (Trimethylsilyloxy)-tricyan-methans mit den Alkoholen in Gegenwart der Mineralsäuren und die nachfolgende Hydrolyse unmittelbar hintereinander in ein und demselben Reaktionsgefäß vornimmt.

**Claims**

1. Process for the preparation of tartronic acid esters of the formula

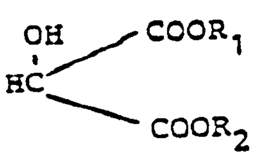

(I)

in which
$R_1$ and $R_2$, independently of one another, represent an optionally substituted alkyl or cycloalkyl radical, characterised in that (trimethylsilyloxy)tricyanomethane is reacted, in the presence of mineral acids, with alcohols of the formula $R_1OH$ and $R_2OH$, in which $R_1$ and $R_2$ have the meaning indicated above, and the product of reaction is hydrolysed.

2. Process according to Claim 1, characterised in that the alcohols $R_1OH$ and $R_2OH$ are employed in an amount of at least 3 mol per mol of (trimethylsilyloxy)tricyanomethane.

3. Process according to Claim 1 and 2, characterised in that the reaction of the (trimethylsilyloxy)tricyanomethane is carried out in a diluent.

4. Process according to Claim 3, characterised in that excess alcohol $R_1OH$ and $R_2OH$ is used as the diluent.

5. Process according to Claim 1 to 4, characterised in that the reaction of the (trimethylsilyloxy)tricyanomethane is carried out at temperatures of −10 to +60°C.

6. Process according to Claim 1 to 5, characterised in that the reaction of the (trimethylsilyloxy)tricyanomethane with the alcohols in the presence of mineral acids, and the subsequent hydrolysis are carried out in immediate succession in one and the same reaction vessel.

**Revendications**

1. Procédé de production d'esters d'acide tartronique de formule

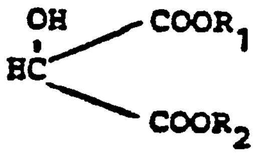

(I)

dans laquelle

$R_1$ et $R_2$ représentent indépendamment l'un de l'autre un reste alkyle ou cycloalkyle éventuellement substitue, caractérisé en ce qu'on fait réagir le (triméthylsilyloxy)tricyanométhane avec des alcools de formules $R_1OH$ et $R_2OH$, dans lesquelles $R_1$ et $R_2$ ont la définition indiquée ci-dessus, en présence d'acides minéraux et on hydrolyse le produit de réaction.

2. Procédé suivant la revendication 7, caractérisé en ce qu'on utilise les alcools $R_1OH$ et $R_2OH$ en une quantité d'au moins 3 moles par mole de (triméthylsilyloxy)-tricyanomethane.

3. Procédé suivant les revendications 1 et 2, caractérisé en ce qu'on conduit la réaction du (trimethylsilyloxy)-tricyanométhane dans un diluant.

4. Procédé suivant la revendication 3, caractérisé en ce qu'on utilise l'alcool $R_1OH$ et $R_2OH$ en excès comme diluant.

5. Procédé suivant les revendications 1 à 4, caractérisé en ce qu'on conduit la réaction du (triméthylsilyloxy)-tricyanométhane à des températures de −10 à +60°C.

6. Procédé suivant les revendications 7 à 5, caractérisé en ce qu'on conduit la réaction du (triméthylsilyloxy)-tricyanométhane avec les alcools en présence d'acides minéraux et on effectue l'hydrolyse ultérieure immédiatement à la suite dans un seul et même récipient de réaction.